# EUROPEAN PATENT APPLICATION

(11) **EP 3 501 539 A1**
(43) Date of publication of application: **26.06.2019**
(21) Application number: 18854307.8
(22) Date of filing: 09.04.2018
(51) Int. Cl.: A61K 39/395, A61K 38/02, A61P 35/00

(54) **APPLICATION OF ANTI-PL2L60 PROTEIN ANTIBODY IN PREPARING ANTI-TUMOR DRUG AND METHOD FOR TREATING TUMORS**

(30) Priority: 07.09.2017 CN 201710800022
(71) Applicant: Shanghai Yifanke Biotechnology Co., Ltd., Shanghai 200000 (CN)
(72) Inventor: GAO, Jianxin, Shanghai 200000 (CN)
(74) Representative: Isern Patentes y Marcas S.L.
(86) International application number: PCT/CN2018/082380
(87) International publication number: WO 2019/047520

(57) **Abstract**

The disclosure relates to the field of medical technology, and provides use of an agent for inhibiting the ectopic expression of a PIWIL2 gene and a protein antibody against the ectopic expression of the PIWIL2 gene in preparation of an anti-tumor drug. The disclosure also provides an anti-tumor pharmaceutical composition and a method for treating a tumor. It has been experimentally proved that, treating human or mouse tumor cells with KAO3 upon inoculation can effectively inhibit the tumorigenesis in a mouse. Furthermore, injecting KAO3 into established lymphoma, breast cancer, lung cancer and cervical cancer can significantly inhibit tumor growth and prolong the survival of a tumor-bearing mouse. The inhibitory effect of KAO3 is associated with KAO3-specific antigens expressed on the surface of a tumor cell. KAO3 induces apoptosis of the tumor cell by blocking the cell cycle in a G2/M phase, inhibiting DNA synthesis and activating a complement. Therefore, the anti-PL2L60 mAb (KAO3) is a potential therapeutic candidate drug for treating a cancer.

## Description

### CROSS-REFERENCE TO RELATED APPLICATIONS

This application claims priority to Chinese patent application No. 201710800022.X filed to the National Intellectual Property Administration on September 07, 2017 and entitled "USE OF ANTI-PL2L60 PROTEIN ANTIBODY IN PREPARATION OF ANTI-TUMOR MEDICINE", which is incorporated herein by reference in its entirety.

### TECHNICAL FIELD

The disclosure belongs to the field of biomedicine, relates to an agent for inhibiting ectopic expression of the *PIWIL2* gene, and use of a protein antibody against the protein products of the ectopically expressed *PIWIL2* gene in preparation of an anti-tumor drug, and in particular relates to use of an antibody against PL2L60, which is a product of intragenically activated *PIWIL2* gene in preparation of an anti-tumor drug, a pharmaceutical composition containing the aforementioned agent or antibody, and a method for treating a tumor.

### BACKGROUND

"Cancer immunotherapy" has good therapeutic effects in various types of cancers since in "Cancer immunotherapy" immunological principles and methods are applied to provide tumor cells with improved immunogenicity and improved sensitivity to killing by an effector cell and stimulate and enhance the body's anti-tumor immune response, and infusion of immune cells and effector molecules into a host body is applied to cooperate with the body's immune system to kill tumors and inhibit tumor growth, and thus is receiving more and more attention

However, targets of cancer immunotherapy are largely individualized, with most targets being distributed only in a few types of cancers, rather than in all types of cancers. Moreover, these molecular targets are also not cancer-specific and are essential for the functions of normal cells. Therefore, the current bottleneck of immunotherapy is the lack of specific and broad-spectrum targets or antigens that are widely expressed in hematopoietic and solid cancers.

### SUMMARY

The objectives of the disclosure include providing use of an agent for inhibiting the ectopic expression of a PIWIL2 gene in preparation of an anti-tumor drug, use of a protein antibody against the protein products of intragenically activated PIWIL2 gene, such as PL2L60 proteins, in preparation of an anti-tumor drug, and a novel anti-tumor drug composition, as well as a method for treating a tumor.

In order to achieve at least one of the aforementioned objectives of the disclosure, the disclosure may employ the following technical solutions.

A first aspect of the disclosure includes providing use of an agent for inhibiting the ectopic expression of a PIWIL2 gene in preparation of an anti-tumor drug.

A second aspect of the disclosure includes providing use of a protein antibody against the ectopic expression of the PIWIL2 gene in preparation of an anti-tumor drug.

The aforementioned anti-tumor drugs include drugs against lymphoma, melanoma, breast cancer, lung cancer, liver cancer, bladder cancer, cervical cancer, prostate cancer, gastric cancer, leukemia, colorectal cancer, colon cancer, ovarian cancer or testicular germ cell tumors.

Furthermore, the protein antibody against the ectopic expression of the PIWIL2 gene as provided by the disclosure is an anti-PL2L protein antibody, and for example may be an anti-PL2L80, anti-PL2L80A, anti-PL2L60, anti-PL2L60A, anti-PL2L50 or anti-PL2L40 protein antibody, and preferably the anti-PL2L60 protein antibody.

In the aforementioned variants, PL2L60 is mainly expressed in precancerous stem cells (pCSCs) as well as various types of human and murine tumor cell lines, and its expression level is much higher than that of a full-length PIWIL2. PL2L60 can promote *in vitro* survival and proliferation of tumor cells by up-regulating STAT3 and BCL2 genes, and can also promote tumorigenesis in coordination with NF-κB, which may represent a common pathway for tumor development in various tissues. It is important that, a peptide derived from PL2L60 can serve as a strong immunogen for various cancer types. The research results show that a PL2L protein, especially an anti-PL2L60 monoclonal antibody (mAb), exhibits a unique ability of directly inducing apoptosis, inhibiting cell proliferation and blocking a cell cycle. Moreover, the PL2L60 protein is one of the targets to which the pCSC-induced naturally occurring tumor immunity (NOTI) is directed to, and may be a common target for cancer immunotherapy.

Furthermore, the anti-PL2L60 protein antibody provided by the disclosure is preferably a KAO3 monoclonal antibody, with the sequence of the KAO3 monoclonal antibody being shown as SEQ ID NO:1.

Furthermore, since the PL2L60 protein can promote the *in vitro* survival and proliferation of tumor cells by up-regulating the STAT3 and BCL2 genes, the anti-PL2L60 protein antibody is an antibody that inhibits activation of the STAT3 or BCL2 gene.

Furthermore, the KAO3 antibody has a potential of preparing a CRA-T cell specific to a PL2L protein.

A third aspect of the disclosure includes providing an anti-tumor pharmaceutical composition which may be composed of an agent for inhibiting the ectopic expression of the PIWIL2 gene and a pharmaceutically-acceptable adjuvant, and may also be composed of a protein antibody against the ectopic expression of the PIWIL2 gene and a pharmaceutically-acceptable adjuvant.

A fourth aspect of the disclosure includes providing a method for treating a tumor, which includes: inhibiting the ectopic expression of the PIWIL2 gene.

In the aforementioned method for treating a tumor, it is preferred to inhibit ectopic expression of the PIWIL2 gene by a KAO3 monoclonal antibody, where the sequence of the KAO3 monoclonal antibody is shown as SEQ ID NO:1.

The tumor may include any one of lymphoma, melanoma, breast cancer, lung cancer, liver cancer, bladder cancer, cervical cancer, prostate cancer, gastric cancer, leukemia, colorectal cancer, colon cancer, ovarian cancer and testicular germ cell tumors.

### The functions and effects of the disclosure include:

In the disclosure it is demonstrated through extensive experiments that, the wide expression of the PL2L protein in various cancer types makes it an ideal broad-spectrum target in immunotherapy for solid and hematopoietic stem cells, and the PL2L60 protein, which is capable of exhibiting the unique ability of directly inducing apoptosis of cancer cells and inhibiting cell proliferation and a cell cycle, has been obtained from various types of PL2L proteins, and meanwhile an anti-PL2L60 mAb (KAO3) against this protein has been developed.

It has been experimentally proved that, treating human or mouse tumor cells with KAO3 upon inoculation can effectively inhibit the tumorigenesis in a mouse. Furthermore, injecting KAO3 into established tumors such as lymphoma, breast cancer, lung cancer and cervical cancer can significantly inhibit tumor growth and prolong the survival of a tumor-bearing mouse. The inhibitory effect of KAO3 is associated with KAO3-specific antigens expressed on the surface of a tumor cell. KAO3 induces apoptosis of the tumor cell by blocking the cell cycle in a G2/M phase, inhibiting DNA synthesis and activating a complement. Therefore, the anti-PL2L60 mAb (KAO3) is a potential therapeutic candidate drug for treating a cancer.

Therefore, the disclosure provides a new basis for preparing an anti-tumor drug using the anti-PL2L60 mAb.

### BRIEF DESCRIPTION OF THE DRAWING

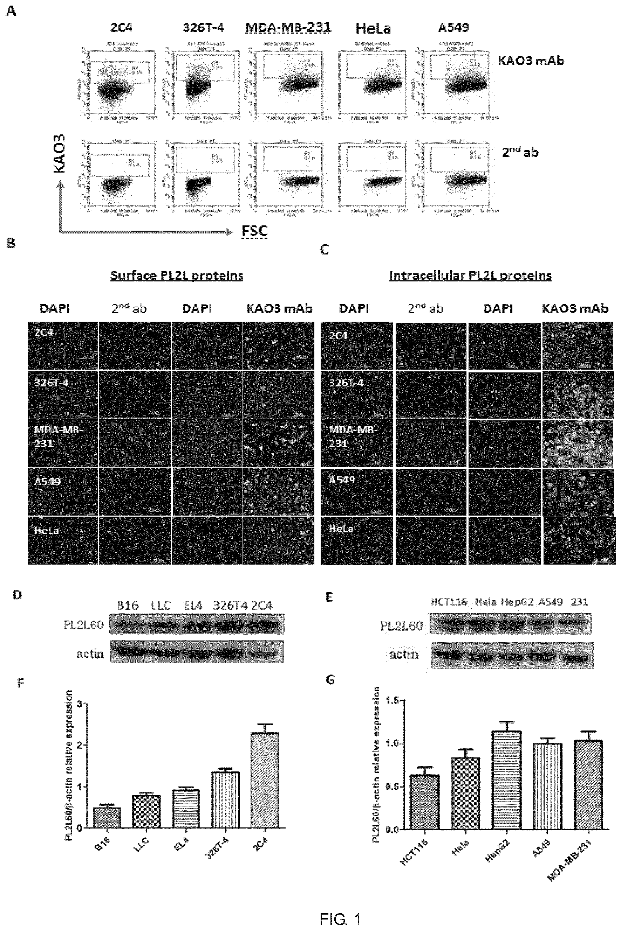

FIG. 1 shows the expression results of the PL2L60 protein in a cancer cell. (A) shows the binding activity results of the anti-PL2L60 monoclonal antibody as determined by a flow cytometer: the expression of the PL2L60 protein on surfaces of plate-cultured cell lines (2C4, 326t-4, MDA-MB-231, A549 and HeLa) is analyzed by the flow cytometer. Cells are harvested by a cells tripper, stained with the PL2L60 mAb under the condition of 4°C for 1 h, then stained with APC-conjugated goat anti-mouse IgM, and analyzed using a BD C6 software; (B) shows the expression of PL2L60 on the surface of a tumor cell as detected by immunofluorescence staining; (C) shows intracellular expression of PL2L60 in a cancer cell line as detected by immunofluorescence staining; (D) and (E) are immunoblotting results: expression of the PL2L60 protein in cancer cells in a plate for Western blotting analysis. Mouse B16 cell lines include LLC, E14, 326t-4 and 2C4; human cell lines include HCT116, HeLa, HepG2, A549 and MDA-MB-231; and (F-G): quantitative analysis of the PL2L60 protein expression in mouse and human tumor cell lines.

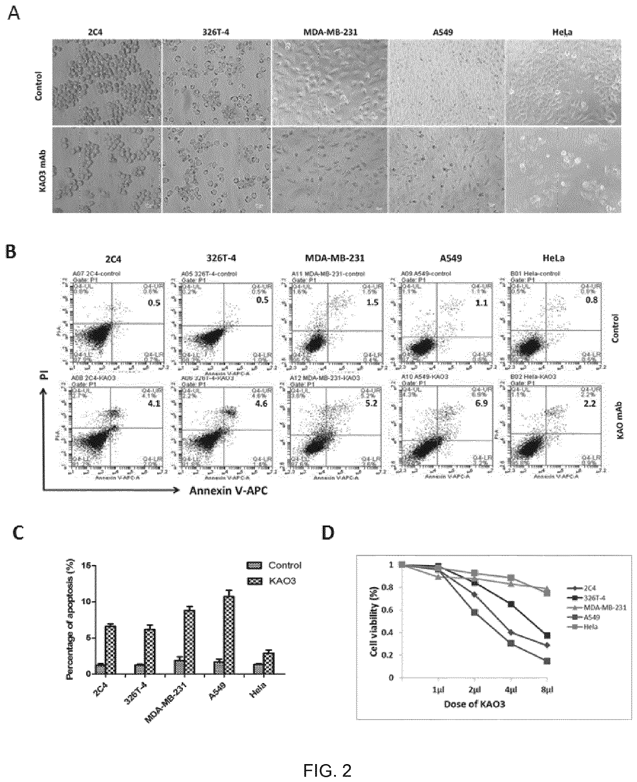

FIG. 2 shows that the anti-PL2L60 mAb KAO3 inhibits proliferation of cancer cells and induces apoptosis *in vitro.* (A) shows the morphology of cancer cells as observed under a phase contrast microscope 48 h after the cancer cells are treated with mAb KAO3, where the bar represents 25 µm; (B) shows the analysis of the cancer cells by flow cytometry 48 h after the cancer cells are treated with the PL2L60 monoclonal antibody; (C) shows the inhibitory effect of the anti-PL2L60 mAb on the cancer cells at the end of the 48 h culturing; (D) shows a summary of the dose-dependent effect of a mAb KAO3 supernatant (µm/ml) on apoptosis induction in cancer cells.

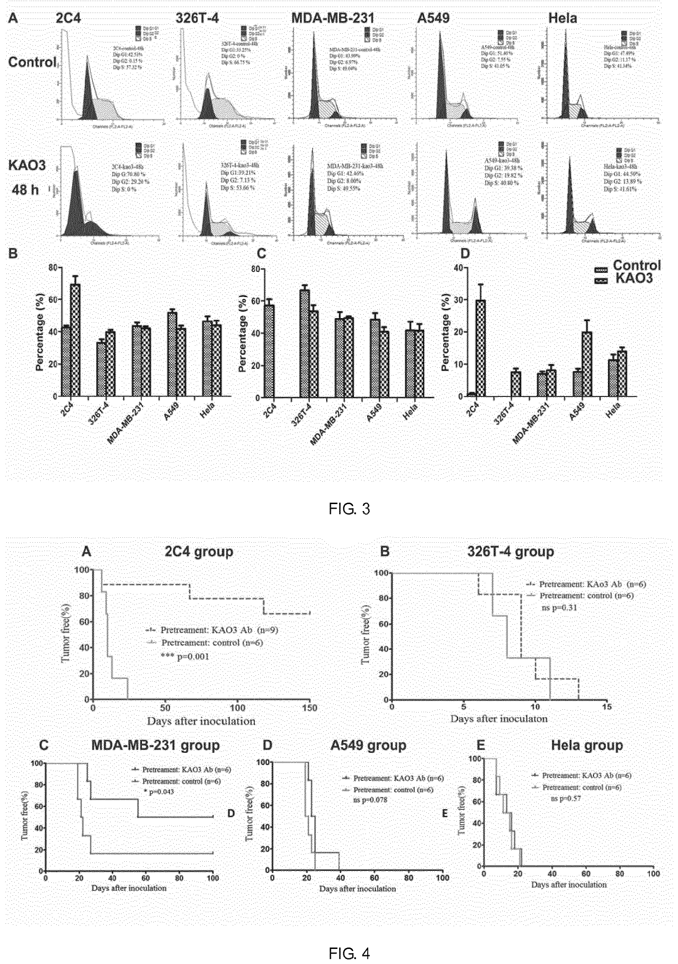

FIG. 3 is a graph showing the cell cycle distribution. (A) is a graph showing the cell cycle distribution of 2C4, 326T-4, MDA-MB-231, A549 and HeLa cell lines which have been treated with KAO3; and (B) shows comparison of the proportion of cells in the G0/G1 phase between a control group and a treatment group. (C) shows the proportion of cells in the S phase; and (D) shows the proportion of cells in the G2/M phase.
FIG. 4 shows the effect of pre-treating cancer cells with KAO3 on tumor incidence. (A)-(E) are the tumor incidences in 2C4, 326T-4, MDA-MB-231, A549 and HeLa cell lines after the KAO3 pretreatment and IgG control pretreatment, respectively, where * represents p < 0.05, and *** represents p < 0.001.

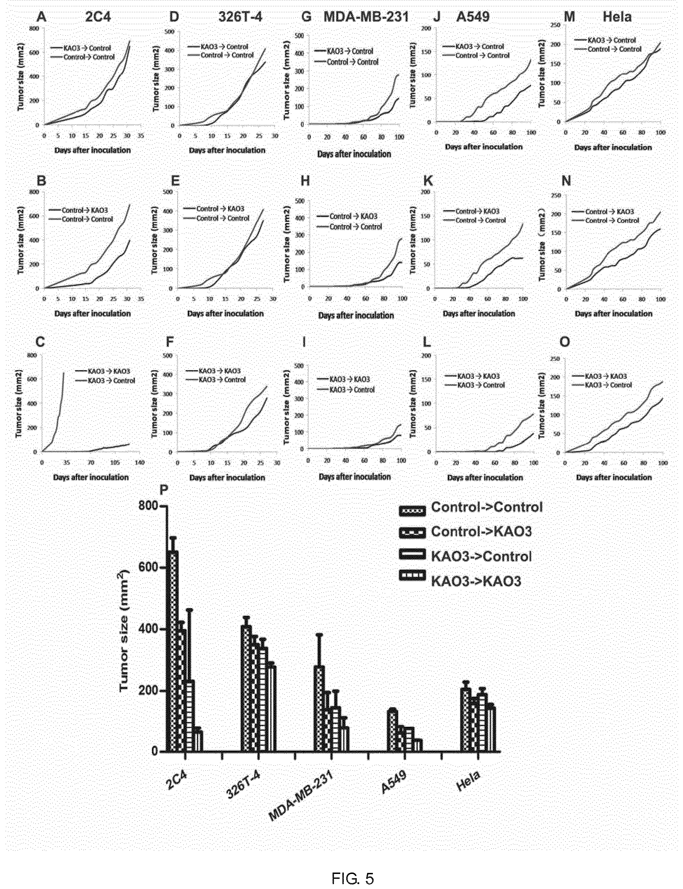

FIG. 5 shows the effects of KAO3 treatment on the tumor-bearing mice at different stages. (A, D, G, J and M) show tumor sizes of mice which are inoculated with cancer cells pretreated with a KAO3 culture supernatant or a medium (R10F)control and then subjected to treatment by the control, where A: 2C4; D: 326T-4; G: MDA-MB-231; J: A549; M: HeLa; (B, E, H, K, and N) show tumor sizes of mice which are inoculated with cancer cells pretreated with the medium (R10F) control and then subjected to treatment by KAO3 or the control, where B 2C4; E: 326T-4; H: MDA-MB-231; K: A549; N: HeLa; (C, F, I, L, O) show tumor sizes of mice which are inoculated with cancer cells pretreated with KAO3 and then subjected to treatment by KAO3 or the control, where C: 2C4; F: 326T-4; I: MDA-MB-231; L: A549; O: HeLa; and (P) shows sizes of tumor cells after different treatments, where 2C4: 35 days; 326T-4: 30 days; MDA-MB-231: 100 days; A549: 100 days; HeLa: 100 days.

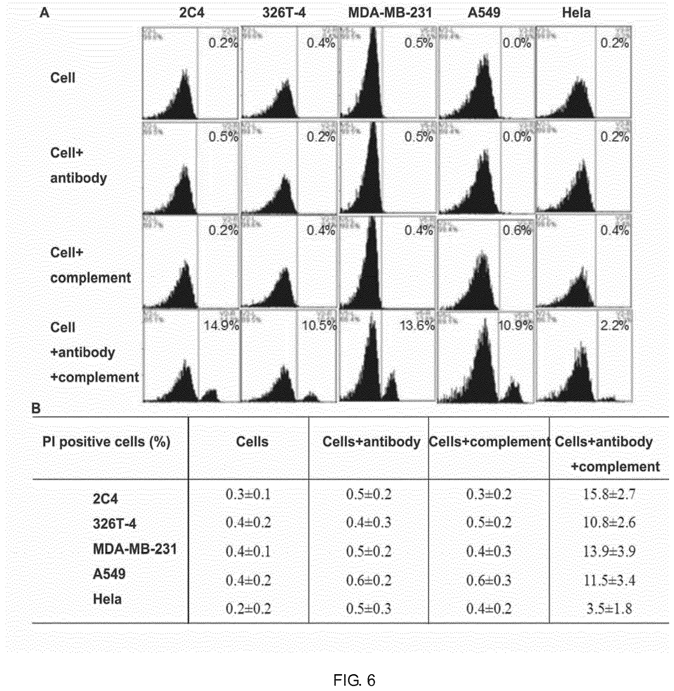

FIG. 6 shows the analysis results of a complement-dependent cytotoxicity (CDC) experiment. The CDC experiment is carried out in the 5 cells mentioned in the disclosure, where (A) is a histogram of PI positive cells. (B) shows the statistical analysis of the PI positive cells. The percentage of dead cells is positively correlated with the anti-tumor efficacy of the anti-PL2L60 mAb. Mouse pCSCs 2C4 and the human breast cancer cell line MDA-MB-231 show the strongest oncolytic effect in the CDC experiment, and the mouse lymphoma cell line 326T-4 and human lung cancer cell line A549 show the second strongest oncolytic effect. The human cervical cancer cell line HeLa has the weakest CDC effect.

### DETAILED DESCRIPTION

In order to make the objectives, technical solutions and advantages of the embodiments of the disclosure more apparent, the technical solution of the embodiments of the disclosure will be described clearly and completely below. If no specific conditions are specified in the embodiments, the embodiments will be carried out according to conventional conditions or the conditions recommended by the manufacturer. All of the used agents or instruments which are not specified with the manufacturer are conventional commercially-available products.

The disclosure will be described in detail below with reference to the embodiments and the accompanying drawings. However, the following embodiments should not be construed as limiting the scope of the disclosure.

The disclosure relates generally to use of an agent for inhibiting the ectopic expression of a PIWIL2 gene in preparation of an anti-tumor drug, and use of a protein antibody against the ectopic expression of the PIWIL2 gene in preparation of an anti-tumor drug.

The aforementioned PIWIL2 is usually expressed in the testis, but can also be activated in somatic cells after DNA damage and then promote DNA repair by remodeling chromatins, and thus it plays a key role in self-renewal and maintenance of a stem cell. Ectopic expression of the PIWIL2 gene has been observed in a variety of primary tumors and tumor cell lines, including breast cancer, lung cancer, liver cancer, bladder cancer, cervical cancer, prostate cancer, gastric cancer, leukemia, colorectal cancer, colon cancer, ovarian cancer and testicular germ cell tumors.

PIWIL2 can promote tumorigenesis by regulating several signal transduction pathways, and inhibits apoptotic death of tumor cells by activation of a Stat3/Bcl-XL pathway. However, most of the PIWIL2-specific antibodies obtained commercially are unable to identify a full-length PIWIL2 from its variants. It should be noted that, in primary breast cancers and cervical cancers, the full-length PIWIL2 protein is mainly detected in apoptotic tumor cells, but not in living tumor cells. In contrast, a PIWIL2 variant, the PL2L protein (e.g., PL2L60) are abundantly detected in various types of tumor tissues and tumor cell lines, indicating that the tumorigenic function of PIWIL2 is mainly mediated by the PIWIL2 variant.

There are many PIWIL2 variants, including PL2L80, PL2L80A, PL2L60, PL2L60A, PL2L50 and PL2L40, etc. Some variants appear to be transcribed by an intragenic promoter rather than a canonical promoter. Although the full-length PIWIL2 can mediate DNA repair, serve as a barrier gene against initiation of tumorigenesis, and promote apoptotic cell death in tumor tissues, its variants such as PL2L60 and PL2L60A can promote tumorigenesis. In the aforementioned variants, PL2L60 is mainly expressed in precancerous stem cells (pCSCs) as well as various types of human and murine tumor cell lines, and its expression level is much higher than that of a full-length PIWIL2. In an *in vitro* experiment, PL2L60 promotes survival and proliferation of tumor cells by upregulating the STAT3 and BCL2 genes. It can also be coordinated with NF-κB to promote tumorigenesis, which may represent a common pathway for tumor development in various types of tissues. It is important that, a peptide derived from PL2L60 can serve as a strong immunogen for targeting various types of cancers. Moreover, PL2L60 is also detected in mouse testicular cells, indicating that it plays a role in gametogenesis or development.

The inventor has found that, a catabolic-activation product of the PIWIL2 gene-the PIWIL2-like (PL2L60) protein, is widely expressed in various hematopoietic and solid tumors and mediates tumorigenesis by promoting tumor cell proliferation and inhibiting apoptosis.

Therefore, tumors can be treated by inhibiting the ectopic expression of the PIWIL2 gene. That is, an agent for inhibiting the ectopic expression of a PIWIL2 gene or a protein antibody against the ectopic expression of the PIWIL2 gene can be used to prepare an anti-tumor drug.

Alternatively, the protein antibody against the ectopic expression of the PIWIL2 gene involved in the disclosure is a PL2L protein antibody, and for example may include at least one of anti-PL2L80, anti-PL2L80A, anti-PL2L60, anti-PL2L60A, anti-PL2L50, and anti-PL2L40 protein antibodies.

Preferably, the protein antibody against the ectopic expression of the PIWIL2 gene involved in the disclosure is the anti-PL2L60 protein antibody.

The inventor of the disclosure mainly develops three monoclonal antibodies (mAbs) against PIWIL2: mAb KAO1, mAb KAO2, and mAb KAO3. In an immunohistochemical staining assay and a Western blot analysis, the mAb KAO2 and mAb KAO3 have stronger affinity to PL2L60 than that of the mAbKAO1, but since the mAbKAO3 is specific to PL2L60, it is considered a potential therapeutic candidate drug for treating a cancer. Therefore, the anti-PL2L60 protein antibody used by the disclosure is preferably a KAO3 monoclonal antibody, with the sequence of the KAO3 monoclonal antibody being shown as SEQ ID NO:1.

Furthermore, the anti-PL2L60 protein antibody involved in the disclosure may also be an antibody that inhibits activation of the STAT3 or BCL2 gene.

Furthermore, the disclosure also provides an anti-tumor pharmaceutical composition, which for example may be composed of the aforementioned agent for inhibiting the ectopic expression of the PIWIL2 gene and a pharmaceutically-acceptable adjuvant, and may also be composed of the aforementioned protein antibody against the ectopic expression of the PIWIL2 gene and a pharmaceutically-acceptable adjuvant.

The pharmaceutically acceptable adjuvant refers to excipients and additives used in drug manufacturing and prescription formulating. It is a substance contained in a pharmaceutical preparation other than the active ingredient. In addition to acting as an excipient, acting as a carrier and improving stability, the pharmaceutical adjuvant also has important functions such as hydrotrope-solubilization, and sustained-release and controlled-release.

Alternatively, the pharmaceutical adjuvant for example may include at least one of a filler, a diluent, a wetting agent, a binder, a disintegrant, a lubricant, a film coating material, a drop pill matrix, and a condensed liquid. Particularly, reference can be made to starch, dextrin, lactose, microcrystalline cellulose, sugar alcohol, ethanol, adhesive cement, polyethylene glycol, methyl cellulose, sodium carboxymethyl starch, magnesium stearate, fine powder silica gel, talcum powder and triethyl citrate.

The antibody contained in the pharmaceutical composition can be referred to the aforementioned anti-PL2L protein antibody, preferably a KAO3 monoclonal antibody. Also, the anti-PL2L protein antibody may also be a therapeutic antibody that targets to PL2L60 and/or inhibits activation of STAT3 and BCL2.

It should be noted that, the amino acid sequence of the antibody may include a sequence as shown in SEQ ID NO: 1 and/or a derived sequence which is obtained through substitutions and/or deletions and/or additions of multiple amino acid residues and has the same biological activity as SEQ ID NO: 1.

The disclosure also provides a method for treating a tumor, which includes: inhibiting the ectopic expression of the PIWIL2 gene. Preferably, the ectopic expression of the PIWIL2 gene is inhibited by the aforementioned KAO3 monoclonal antibody. The tumor referred to herein includes any one of lymphoma, melanoma, breast cancer, lung cancer, liver cancer, bladder cancer, cervical cancer, prostate cancer, gastric cancer, leukemia, colorectal cancer, colon cancer, ovarian cancer and testicular germ cell tumors.

The experimental methods in the following embodiments which are not specified with specific conditions are generally carried out according to conventional conditions or according to the conditions recommended by the manufacturer. The percentages and parts of the substance are calculated by volume, unless otherwise stated.

The materials used in the disclosure are as follows.

Severe combined immunodeficiency (SCID) mice: mice of 8-12 weeks old are used, these mice being fed in animal harmless facilities.

Human cell lines: the human breast cancer cell line MDA-MB-231, the human lung cancer cell line A549 and the human cervical cancer cell line HeLa, which are all available from American Type Culture Collection (ATCC, Manassas, VA, USA). The cells were maintained in DMEM (Gibco) supplemented with 10% fetal bovine serum (Gibco) and 0.1 mg/ml penicillin-streptomycin (Gibco).

Mouse lymphoma cell lines 2C4 and 326T-4 are self-made in the laboratory: cells are placed in a humidified incubator containing 5% CO₂ (v/v), and maintained in R10F (RPMI 1640 plus 10 mmol/L fetal bovine serum, supplemented with 5 mmol/L glutamine, 50 mmol/L 2-methyl acetophenone, 100 U/mL penicillin and 100 mg/ml streptomycin) at 37°C.

The anti-PL2L60 monoclonal antibody (KAO3mAb, isoform IgM) is self-made in the laboratory according to the preparation procedure of a monoclonal antibody, and has a sequence as shown in SEQ ID NO:1.

For reference, the preparation process of the monoclonal antibody in the disclosure for example may include:

### (1) Immunizing Animals

Immunizing animals is a process in which a mouse is immunized with an antigen of interest such that the mouse produces a sensitized B lymphocyte. Female Balb/c mice of 6-8 weeks old are generally selected and subjected to inoculation according to a pre-established immunization protocol. The antigen enters a peripheral immune organ through blood circulation or lymph circulation and thus stimulates cloning of corresponding B lymphocytes, such that the B lymphocytes are activated, proliferated, and differentiated into sensitized B lymphocytes.

### (2) Cell fusion

The mice are sacrificed by a carbon dioxide gas, and the spleens are removed through aseptical operations, and subjected to extrusion grinding in a dish to prepare a suspension of spleen cells. The prepared homologous myeloma cells are mixed with mouse spleen cells in a certain ratio, and added with a fusogen polyethylene glycol. Under the action of polyethylene glycol, various lymphocytes can be fused with myeloma cells to form hybridoma cells.

### (3) Selective Culture

The purpose of selective culture is to screen out fused hybridoma cells, where optionally, for example a HAT selective medium can be employed. In the HAT medium, the unfused myeloma cells are died since they are unable to synthesize DNA by means of a salvage pathway due to the lack of a hypoxanthine-guanine-phosphoribosyltransferase. Although unfused lymphocytes have the hypoxanthine-guanine-phosphoribosyltransferase, they cannot survive *in vitro* for a long term and thus gradually die. Only the fused hybridoma cells can survive and proliferate in the HAT medium since they obtain the hypoxanthine-guanine-phosphoribosyltransferase from spleen cells and have the infinite multiplication property of myeloma cells.

### (4) Screening and Cloning of Hybridoma Positive Clones

Only a small number of the hybridoma cells grown in the HAT medium are cells that secrete predetermined specific monoclonal antibodies, and therefore screening and cloning must be performed. Optionally, clonal culture of hybridoma cells can be performed for example by a limiting dilution method. Using sensitive, rapid, and specific immunological methods, positive hybridoma cells capable of producing the desired monoclonal antibody are screened and subjected to clonal expansion. After the immunoglobulin type, subclass, specificity, affinity, epitope for antigen recognition and molecular weight of the monoclonal antibody secreted by the cells are comprehensively identified, the cells are cryopreserved in time.

### (5) Large-scale preparation of monoclonal antibodies

Large-scale preparation of monoclonal antibodies can be conducted by an animal *in vivo* induction method and an *in vitro* culture method. The *in vivo* induction method includes: taking a Balb/c mouse, and first pretreating the mouse with intraperitoneal injection of 0.5 ml liquid paraffin or pristane. After 1-2 weeks, the mouse is intraperitoneally inoculated with hybridoma cells. The hybridoma cells are proliferated in the abdominal cavity of mouse and produce and secrete monoclonal antibodies. After about 1-2 weeks, it can be seen that the abdomen of the mouse is enlarged. A large amount of monoclonal antibodies can be obtained by withdrawing the ascites fluid with a syringe. Furthermore, the *in vitro* culture method includes: culturing the hybridoma cells in a culture flask. During the culture process, the hybridoma cells produce and secrete monoclonal antibodies, and the culture supernatant is collected and centrifuged to remove cells and debris thereof, so as to obtain the desired monoclonal antibodies.

The inventor of the disclosure has developed three monoclonal antibodies (mAbs) against PIWIL2 in total: mAb KAO1, mAb KAO2, and mAb KAO3. Since mAbKAO3 is specific to PL2L60, the KAO3 monoclonal antibody is preferably used in the following analysis methods.

The analysis methods used in the disclosure and the experimental results will be described in detail below.

### I. Analysis Methods

### Analysis by flow cytometer

For reference, for example the cancer cells can be dissociated with 0.25% trypsin-EDTA (1mM; Invitrogen) for 1-3 min, washed with a cell sorting buffer (PBS containing 1% fetal bovine serum), and then incubated with anti-PL2L60 mAbs at 4°C for 1 h. The cells are incubated with phycoerythrin-conjugated goat anti-mouse IgM (1:250 dilution; Bioligend) at 4°C for 30 min. After the final wash, the cells are resuspended in PBS containing 1% FBS and analyzed by flow cytometry (BD, San Jose, CA, USA). Cell-surface and intracellular immunofluorescence staining.

For surface staining, tumor cells are collected and resuspended in PBS at a cell concentration of 5 x 10⁶/ml, and are added into a 96-well plate with 0.2 mL of cell suspension (1 x 106cells/well). The cells are rotated (1000 rpm, 5 min) and the supernatant is discarded. The anti-PL2L60 monoclonal antibody KAO3 is added into 100 µL PBS and rotated for 5 s. The sample is incubated at 4°C for 30 min. The cells are washed with PBS twice. The supernatant is discarded, and 100 µl of PBS containing 1% paraformaldehyde is added into each well to fix the cells.

For intracellular staining, the tumor cells cultured on a cover slip are fixed in 2% paraformaldehyde for 20 min, then washed, and then blocked with PBS containing 1% bovine serum albumin for 30 min. Cells are incubated with anti-PL2L60 mAb (KAO3, 1:100 dilution) in 1% bovine serum albumin at room temperature. After 1 h of incubation, the cells are washed and co-incubated with FITC goat anti-mouse antibody (IgM, Bioligend). The cell nucleus is stained with a staining reagent such as 4',6-diamidino-2-phenylindole (DAPI, 1:500).

### Western Blot

Prior to harvesting with trypsin, the cell samples are washed in cold PBS twice, and then lysed with a protein extraction reagent. The total protein concentration of the whole cell lysate is determined using a BCA protein assay kit (Beyotime, Shanghai, China), and then the protein is separated using a 12% polyacrylamide gel and transferred onto a polyvinylidene fluoride membrane.

After blocked with 5% bovine serum albumin (BSA) in TBS/Tween 20 (TBST), the cells are incubated with a specific primary antibody at 4°C overnight, and the membrane is washed with TBS-T for 5 min, where the washing is repeated for three times. Thereafter, the membrane is incubated with a horseradish peroxidase-conjugated anti-mouse IgM secondary antibody (Santa Cruz Biotechnology, Santa Cruz, CA, USA) at room temperature for 1 h, followed by washing with TBST for 5 min, where the washing is repeated for three times. The membrane is then analyzed using an ECL chemiluminescence detection system (Bio-Rad).

The following antibodies are used in this study: an anti-PIWIL2 polyclonal antibody (prepared by our group) and images are obtained from Kodak Imaging Station 2000R (Eastman Kodak, USA).

### CCK-8 Analysis

The effect of anti-PL2L60 mAb (KAO3) on cell viability was assessed using CCK-8 assay (Dojindo, Japan). For conducting CCK-8 assay, cells are mixed with concentration gradients (1, 2, 4, and 8 µg/ml) of KAO3 in a final volume of 200 µL medium containing 10% FBS, with the same concentration of IgG being used as a control. The serially diluted PL2L60 antibody or the control IgG is then inoculated onto a flat-bottom 96-well plat at a density of 2 x 10³ cells/well. After incubation for 48 h, 10 µL of CCK-8 reagent is pipetted into each well of a 96-well assay plate (containing 100 µL of fresh phenol red-free medium in the well), and the plate is incubated in a humid atmosphere under 5% CO₂ at 37°C for 1-4 h. The absorbance (A) at 490 nm is then recorded using a Spectra® Max M5 series (Molecular Devices).

The cell viability is calculated as (Aₛₐₘₚₗₑ-A_{blank})/(A_{control}-A_{blank}) x 100%. All experiments are repeated for at least three times and in triplicate for each experiment.

### Apoptosis Assay

After 24 h of treatment with KAO3, cells are harvested and washed twice with pre-cooled PBS. The IgG2a isotype control (Biolegend) is diluted and then incubated with a mixture containing annexin V (Biolegend) and propidium iodide (Sigma) in a binding buffer in the dark for 15 min. Apoptotic cells are detected using annexin V-APC and PI and analyzed using a flow cytometer (BD, C6). Early apoptotic cells are assayed using an Annexin V-APC Apoptosis Assay Kit (Biolegend). An apoptotic cell nucleus is detected by staining with propidium iodide (PI, Sigma). Three independent experiments are performed in total.

### Cell Cycle Analysis

The cells are fixed in frozen 75% ethanol, and subjected to staining for cell cycle analysis in PBS with a propidium iodide (PI) solution containing 100 µg/ml ribonuclease (Tiangen Biotech) and 50 µg/mL PI (Biolegend). The percentage of cells at each stage of the cell cycle is measured by a C6 flow cytometer (BD).

### Anti-Tumor Test

Cells (with a cell concentration of 5 x 10⁶ per 200 µL PBS) are injected into the groins of SCID mice. After tumorigenesis, the tumor-bearing mice are randomly divided into four groups: group 1: control→control group; group 2: control→KAO3; group 3: KAO3→control group; and group 4: KAO3→KAO3. KAO3 or isotype IgG is injected orthotopically for two weeks. The length and width of the tumor are measured with a caliper every 2 days, and the mice are euthanized when the tumor reaches a diameter of 2 cm.

### Complement-Dependent Cytotoxicity (CDC) Analysis

Complement-dependent cytotoxicity (CDC) target cells are inoculated into a plate at a concentration of 5 x 10⁴ cells/well. Test antibodies are added to activated or heat-inactivated (60°C, 30 min) human serum (with a final serum concentration of 25%; Pathway Diagnostics, Dorking, UK) at a specified final concentration.

The plate is incubated at 37°C for 3 h and then added with a cell viability reagent. Triton 1 X-100 is added to the wells containing control cells to establish the maximum lysis control. After incubated at 37°C for 1 h, fluorescence is measured by a fluorescence microscope (Olymplus, Japan).

All experimental data is obtained from at least three independent experiments. Experimental numerical values are expressed as mean ± standard deviation (SD), and the experimental group is compared with a corresponding control by Student's t-test. Three and more groups are compared by one-way analysis of variance (ANOVA). When the p value is less than 0.05, the difference is considered to be significant. * indicates p value < 0.05; ** indicates p value < 0.01; and *** indicates p value < 0.001. Survival analysis is performed using a Kaplan-Meier survival curve, and a significant difference between groups is tested by using a log-rank test, and a correlation coefficient is determined by Spearman analysis.

It should be noted that, the analysis parameters, analysis conditions, the reagents as used, and the like involved in the aforementioned analysis methods can be appropriately adjusted according to the specific conditions and situations of the experiment.

### II. Experimental Results

### Protein Expression in Cancer Cell

By immunohistochemistry, in primary breast cancers and cervical cancers, the full-length PIWIL2 protein is mainly detected in apoptotic tumor cells, but is almost no detectable in living tumor cells. In contrast, a PIWIL2 variant, the PL2L protein (e.g., PL2L60) are abundantly detected in various types of tumor tissues and tumor cell lines, indicating that the tumorigenic function of PIWIL2 may be mainly mediated by the PIWIL2 variant. Thus, the widespread expression of the PL2L protein in various cancer types makes it an ideal broad-spectrum target for solid and hematopoietic stem cell immunotherapy.

To demonstrate this hypothesis, we first investigate the expression of the PL2L protein on the cell surfaces of various types of tumor cell lines using a monoclonal antibody (mAb KAO3) against human and mouse homologous PIWIL2 peptides. In addition to in cytoplasm, the PL2L protein is also detected on the surfaces of tumor cell lines by flow cytometry and fluorescence microscopy (FIGs. 1A-B), including a mouse hematopoietic precancerous stem cell (pCSC) line 2C4, a cancer stem cell (CSC) line 326T-4, a human breast cancer cell line MDA-MB-231, a lung cancer cell line A549 and a cervical cancer cell line HeLa.

Intracellular immunofluorescence analysis (FIG. 1C) shows that the PI2L60 protein is mainly expressed in the cytoplasm of various types of human and mouse tumor cells, and there is no significant difference among different cancer cells. Western blotting data shows that the PL2L60 total protein is highly expressed in various types of human and murine tumor cell lines, and the expression level is the highest in the cytoplasm of pCSCs 2C4 (FIGs. 1D-G).

### Cytotoxicity of KAO3 mAb

In an *in vitro* experiment, PL2L60 can promote survival and proliferation of tumor cells by up-regulating the STAT3 and BCL2 genes, and it can also promote tumorigenesis in coordination with the NF-κB protein. Therefore, we have developed antibodies that inhibit cancer proliferation by producing monoclonal antibodies against PL2L60. There is currently no public report on anti-PL2L60 antibodies that can directly inhibit proliferation and induce apoptosis. In the current study, we have examined whether the newly produced anti-PL2L60 mAb has this ability.

KAO3mAb can induce apoptosis in cancer cells, such as MDA-MB-231, A549, HeLa, 2C4 and 326T-4 (FIG. 2). Before KAO3 treatment, the morphologies of cancer cells in the control group and the experimental group are basically the same, the control cells grow well with a polygonal shape, and the size of the control cells is uniform after 48 h of culture. After 48 h of treatment with the anti-PL2L60mAb KAO3, loss of cell chromatin is observed under a high-power microscope, the number of cells is significantly reduced, the cell morphology is irregular, and some cells become round. Visible nuclear chromosomes and reduced cytoplasmic vacuoles are observed under a high-power microscope (FIG. 2A). The number of semi-independent cells and apoptotic cells in the suspension is increased dramatically (FIGs. 2B and 2C). Specifically, cell viability is reduced after the treatment with the anti-PL2L60 antibody at different doses (1, 2, 4, and 8 µl/well of mAb KAO3 supernatant) for 48 h (FIG. 2D).

These results indicate that the anti-PL2L60 mAb KAO3 can significantly inhibit cell proliferation of five cancer cell lines (FIG. 2D). Collectively, these results indicate that the anti-PL2L60 mAb (KAO3) may induce a cytotoxic activity of cancer cells by inducing an apoptotic pathway.

### Inducement of KAO3 mAb on cell cycle arrest in cancer cells

FIG. 3A shows cell cycle distributions of 2C4,326T-4, MDA-MB-231, A549 and HeLa after the treatment with anti-PL2L60 mAb (KAO3). Compared with the control cells, after treatment with the anti-PL2L60 mAb (KAO3) for 48 h, the proportion of cancer cells in the G0/G1 phase is decreased slightly, and the percentage of cells in the S phase has little change. The number of cells in the G2/M phase is increased. A549 cells have the highest number of cells in the G2/M phase. The proportion of cells in G0/G1 and G2/M phases is increased sharply, and the percentage of cells in the S phase is decreased sharply in MDA-MB-231 and HeLa cells. The 2C4 cells in G0/G1, S and G2/M phases are subjected to the maximal changes. There is no significant change in 326T-4 cells (FIGs. 3B-3D). Briefly, the PI/FACS analysis indicates that the anti-PL2L60 mAb (KAO3) causes significant arrest at the G2/M phase in five cancer cell lines.

### Treatment of xenograft tumors with anti-PL2L60 mAb KAO3

Since the treatment with the anti-PL2L60 monoclonal antibody (KAO3) disrupts cancer cell growth and induces cancer cell apoptosis *in vitro,* it is investigated whether KAO3 can be used to directly inhibit tumor growth *in vivo.* In order to observe the effect of KAO3 on the tumorigenicity of tumor cells, the tumor treatment program is divided into two stages: one is the initial stage of tumorigenesis, and the other is the stage of tumor treatment.

First, human and mouse cancer cells are suspended in the culture supernatant of KAO3 hybridoma or culture medium and then inoculated into SCID mice, and the cells are treated with the isotype IgG to serve as a control. After tumor formation, tumor-bearing mice in different treatment groups are divided into two groups. One group of tumor-bearing mice is injected orthotopically with KAO3 every two days, and the other group is injected with the isotype IgG as a control, and tumor growth is observed.

In the initial stages of tumorigenesis, the tumor incidences of different groups are counted when the average tumor diameter is close to 0.5 cm. We find that the tumorigenesis of pCSCs in SCID mice is almost completely inhibited by the KAO3 mAb. Within 150 days of observation, only 33% of the mice (3/9) are subjected to tumorigenesis on days 10, 67 and 118 after vaccination. In contrast, all mice (100%) in the control group are subjected to tumorigenesis within 3 weeks after vaccination. The tumor incidence of the mice inoculated with KAO3-pretreated MDA-MB-231 cells is 50%, compared with 83% in the control group. The tumor incidence in each of the other three cell lines is 100%, and there is no difference between the KAO3-pretreated group and the control group (FIGs. 4A-4E).

Further analysis of tumor growth kinetics shows that, one tumor growth rate from KAO3-pretreated pCSCs is relatively slower than the tumor growth rate from the pCSCs in the control (treated with the medium) (FIG. 5A). The tumors in the control group are significantly inhibited by mAb after injection of 50 µl KAO3 mAb supernatant (FIG. 5B). The tumors from the pCSCs pretreated with the KAO3 mAb and grown on days 67 and 118 are almost completely inhibited by mAb (FIG. 5C). The results show that, mAb KAO3 can effectively prevent tumorigenesis of pCSCs and inhibit the growth of established tumors. The same treatment on mouse hematopoietic stem cells (CSCs; clones of 326T-4) and 326T-4 also result in varying degrees of tumorigenicity or tumor growth inhibition in SCID mice (FIGs. 5D-5F).

Since the mAb KAO3 also recognizes a human PL2L60 protein, similar result is observed when the human breast cancer cell lines MDA-MB-231, the human lung cancer cell lines A549 and the human cervical cancer cell lines HeLa are treated with the culture supernatant of hybridoma KAO3 by the same method as that used for treating the 2C4 pCSC line. That is, KAO3 mAb can also effectively inhibit tumorigenesis of human cancer cells and growth of established tumors in SCID mice (FIGs. 5G-5P). The results indicate that mAb KAO3 can effectively kill or inhibit cancer cells in humans and mice.

The function of the mAb KAO3 appears not to be limited to the two kinds of tumors, as the same treatment on the murine hematopoietic stem cells (CSCs; clones of 326T-4), the human lung cancer cell lines A549 and human cervical cancer cell lines HeLa also results in inhibition to varying degrees of tumorigenicity or tumor growth in the SCID mice. These results confirm our findings that KAO3 has a higher therapeutic efficacy than that of the control in reducing tumor growth, it is shown that KAO3 can inhibit the *in vivo* tumor growth in human and mice.

### Complement-dependent cytotoxicity (CDC) of mAb KAO3

The anti-tumor therapeutic effect is positively correlated with the expression level of the PL2L60 protein on the surfaces of CDC tumor cells, since in the presence of an *in vitro* complement, mAb can kill tumor cells. Therefore, the PL2L60 protein is also a suitable target for passive cancer immunotherapy.

The results show that, the mouse pCSC line 2C4 and the human breast cancer cell line MDA-MB-231 show the strongest oncolytic effect in the CDC experiment, and the mouse lymphoma cell line 326T-4 and human lung cancer cell line A549 show the second strongest oncolytic effect. The human cervical cancer cell line HeLa has the weakest CDC effect (FIGs. 6A-B). These results are consistent with the expression level of the PL2L60 protein on the surfaces of cancer cells. It further indicates that the anti-PL2L60 mAb KAO3 is a target of the PL2L60 protein on the surfaces of the tumor cells, and KAO3 exerts its anti-tumor effect through a CDC-dependent mechanism.

In view of the above, the development of therapeutic antibodies that target to PL2L60 and/or inhibit STAT3 and BCL2 activation has enormous potential to eliminate tumors.

In this study, we use the mAb KAO3 developed by our laboratory to test whether the PL2L protein is a common target for cancer immunotherapy. Differential expression of the mAb KAO3 in inhibiting tumorigenesis and tumor growth appears to be associated with expression of a surface KAO3-specific antigen (i.e., the PL2L protein) among cancer cell lines (FIGs. 1A-1B), but not associated with intracellular expression (FIGs. 1C-1G), since the ability of KAO3 mAb of inhibiting tumorigenesis is associated with the percentage of surface KAO3+ cells. Compared with other lines, the HeLa and 326T-4 cell lines contain less KAO3+ cells and are less sensitive to mAb KAO3 treatment (FIG. 5). Therefore, the therapeutic efficacy of mAb KAO3 is determined by the surface expression level of the PL2L protein.

The mechanism by which the anti-PL2L60 antibody inhibit tumors in vivo is still unclear. No published study has demonstrated that the anti-PL2L60 antibody directly induces apoptosis in cancer cells in a clinical setting, and thus it is unclear whether this antibody can directly inhibit tumor cell proliferation. In this study, the KAO3 mAb treatment inhibits proliferation of human and mouse cancer cells and significantly increase the percentage of cells in the G2/M phase of the cell cycle in a dose-dependent manner, and particularly the percentage of 2S4 cells in the S phase is significantly reduced.

We study the anti-tumor effect of KAO3, which has a strong inhibitory effect on the cell growth of cancer cells. We demonstrate that KAO3 induces cell cycle arrest in the G2/M phase in a dose-dependent manner, and the cell cycle arrest in turn develops into apoptosis (FIGs. 2-3), which may be associated with microtubule depolymerization or inhibition of nuclear translocation of NF-κB and STAT3, and in turn inhibits the transcription activity. In the tumor xenograft model, KAO3 effectively delays tumor growth (FIGs. 4-5). Our results provide direct evidences that our anti-PL2L60 antibody inhibits tumor cell proliferation and induces apoptosis.

Results indicate that, the PL2L protein is an effective target for cancer immunotherapy. We first discovery that the mAb KAO3 can recognize surface PL2L proteins expressed on various types of tumor cell lines, including for example lymphoma, melanoma, breast cancer, lung cancer, cervical cancer, liver cancer, bladder cancer, prostate cancer, gastric cancer, leukemia, colorectal cancer, colon cancer, ovarian cancer or testicular germ cell tumors. The mAb KAO3 can effectively inhibit tumorigenesis in human and mice *in vivo* and inhibit tumor cell proliferation *in vitro,* by inducing cell cycle arrest in the G2/M phase and complement activation. Its inhibition effect is closely related to the number of surface KAO3 + cells in tumor cell lines.

The low expression of PL2L60 on the cell surface may have a significant response to the KAO3 treatment *in vitro* and *in vivo,* which is related to the stem/progenitor cell properties of these cancer cells. The pCCs having high level expression of PL2L60 are more sensitive to the tumorigenic inhibition by the mAb KAO3, as the inhibition effect of the KAO3 mAb is more effective in tumor stem/progenitor cells than that in non-stem-cell cancer cells. It should be noted that, cancer cells bearing less PL2L60 in the cell lines are associated with lower tumor growth rates. It indicates that PL2L60-bearing cancer cells may represent stem/progenitor cancer cells at various developmental stages, or represent the only target of the KAO3 mAb. These results demonstrate that the PL2L protein plays a key role in tumorigenesis and thus is a common effective target for cancer immunotherapy. Our findings provide a new venue for cancer treatment by cancer immunotherapy.

In view of the above, PL2L60 may be a promising target antigen for cancer treatment. All data indicate that, as a PL2L60-specific epitope, the KAO3 monoclonal antibody has a significant anti-tumor activity.

The basic principles, main features, and advantages of the disclosure are shown and described above. It should be understood by those skilled in the art that, the disclosure is not limited by the aforementioned embodiments. The aforementioned embodiments and the description only illustrate the principle of the disclosure. Various changes and modifications may be made to the disclosure without departing from the spirit and scope of the disclosure. Such changes and modifications all fall within the claimed scope of the disclosure. The scope of the disclosure is defined by the appended claims and their equivalents.

### INDUSTRIAL APPLICABILITY

Thus, the widespread expression of the PL2L protein provided by the disclosure in various cancer types makes it an ideal broad-spectrum target for solid and hematopoietic stem cell immunotherapy. The PL2L60 protein, which is capable of exhibiting the unique ability of directly inducing apoptosis of cancer cells and inhibiting cell proliferation and a cell cycle, has been obtained from various types of PL2L proteins, and especially the anti-PL2L60 mAb (KAO3) has been developed. Treating human or mouse tumor cells with KAO3 upon inoculation can effectively inhibit the tumorigenesis in a mouse. Furthermore, injecting KAO3 into established tumors such as lymphoma, breast cancer, lung cancer and cervical cancer can significantly inhibit tumor growth and prolong the survival of a tumor-bearing mouse. The inhibitory effect of KAO3 is associated with KAO3-specific antigens expressed on the surface of a tumor cell. KAO3 induces apoptosis of the tumor cell by blocking the cell cycle in a G2/M phase, inhibiting DNA synthesis and activating a complement. Therefore, the anti-PL2L60 mAb (KAO3) is a potential therapeutic candidate drug for treating a cancer.

序列表

<110>  上海易范科生物科技有限公司

<120>  抗 PL2L60 蛋白抗体在制备抗肿瘤药物中的应用及治疗肿瘤的方法

<130>  2017

<160>  1

<170>  SIPOSequenceListing 1.0

<210>  1
<211>  1382
<212>  PRT
<213>  KAO3

<400>  1
Ala Ile Val Met Thr His Thr Pro Ser Ser Leu Val Thr Ala Gly Gly
1               5                   10                  15
Leu Val Thr Met Ser Cys Leu Ser Ser Gly Ala Leu Leu Leu Ser Gly
            20                  25                  30
Ala Gly Ala Thr Leu Thr Thr Gly Gly Leu Pro Gly Pro Pro Leu Leu
        35                  40                  45
Leu Ile Thr Thr Ala Ser Thr Ala Gly Ser Gly Val Pro Ala Ala Pro
    50                  55                  60
Thr Gly Thr Ala Pro Thr Leu Thr Ile Ser Ser Val Gly Ala Gly Ala
65                  70                  75                  80
Leu Ala Val Thr Thr Cys Gly Ala Ala Thr Ser Thr Pro Leu Thr Pro
                85                  90                  95
Gly Thr Thr Leu Gly Leu Leu Gly Val Thr Leu Leu Val Ser Gly Pro
            100                 105                 110
Gly Ile Leu Leu Pro Ser Thr Leu Thr Leu Ser Leu Thr Cys Pro Ser
        115                 120                 125
Gly Pro Ser Gly Met Gly Val Gly Thr Ile Ala Gly Thr Ile Gly Pro
    130                 135                 140
Ser Gly Leu Gly Thr Leu Ala His Ile Thr Thr Ala Ala Ala Leu Thr
145                 150                 155                 160
Thr Ala Pro Ser Leu Leu Ser Gly Leu Thr Ile Ser Leu Ala Thr Ser
                165                 170                 175
Ala Ala Gly Val Pro Leu Leu Ile Thr Ser Val Ala Thr Ala Ala Thr
            180                 185                 190
Ala Ala Thr Thr Thr Cys Ala Thr Thr Thr Cys Ala Ala Ala Gly Ala
        195                 200                 205
Thr Ala Thr Ala Met Ala Thr Thr Gly Gly Gly Thr Ser Val Thr Val
    210                 215                 220
Ser Ser Ser Gly Thr Ser Gly Ser Val Thr Pro Thr Ala Leu Leu Pro
225                 230                 235                 240
Ser Gly Thr Leu Ser Leu Thr Cys Ser Pro Ser Gly Pro Ser Leu Ser
                245                 250                 255
Thr Ser Gly Met Gly Val Gly Thr Ile Ala Gly Pro Ser Gly Leu Gly
            260                 265                 270
Thr Leu Ala His Ile Thr Thr Ala Ala Ala Leu Thr Thr Ala Pro Ser
        275                 280                 285
Leu Leu Ser Gly Leu Thr Ile Ser Leu Ala Thr Ser Ala Ala Gly Val
    290                 295                 300
Pro Leu Leu Ile Thr Ser Val Ala Thr Ala Thr Thr Thr Cys Ala Ala
305                 310                 315                 320
Ala Gly Ala Thr Ala Thr Ala Met Ala Thr Thr Gly Gly Gly Thr Ser
                325                 330                 335
Val Thr Val Ser Ser Ala Ile Leu Leu Thr Gly Ser Pro Ala Ser Leu

            340                 345                 350
Ala Val Ser Leu Gly Gly Ala Ala Thr Ile Ser Thr Ala Ala Ser Leu
        355                 360                 365
Ser Val Ser Thr Ser Gly Thr Ser Gly Thr Ser Thr Met His Thr Ala
    370                 375                 380
Gly Gly Leu Pro Gly Gly Pro Pro Ala Leu Leu Ile Thr Leu Leu Ile
385                 390                 395                 400
Thr Leu Val Ser Ala Leu Gly Ser Gly Val Pro Ala Ala Pro Ser Gly
                405                 410                 415
Ser Gly Ser Gly Thr Ala Pro Thr Leu Ala Ile His Pro Val Gly Gly
            420                 425                 430
Gly Ala Ala Ala Thr Thr Thr Cys Gly His Ile Ala His Ala Gly Leu
        435                 440                 445
Thr Ala Ser Gly Gly Gly Pro Ser Thr Leu Ala Gly Val Thr Leu Leu
    450                 455                 460
Val Cys Gly Pro Gly Ile Leu Leu Pro Ser Gly Thr Leu Ser Leu Thr
465                 470                 475                 480
Cys Ser Pro Ser Gly Pro Ser Leu Ser Thr Ser Gly Met Gly Val Gly
                485                 490                 495
Thr Ile Ala Gly Pro Ser Gly Leu Gly Leu Gly Thr Leu Ala His Ile
            500                 505                 510
Thr Thr Ala Ala Ala Leu Thr Thr Ala Pro Ser Leu Leu Ser Gly Leu
        515                 520                 525
Thr Ile Ser Leu Ala Thr Ser Ala Ala Gly Val Pro Leu Leu Ile Thr
    530                 535                 540
Ser Val Ala Thr Ala Ala Thr Ala Ala Thr Ala Thr Thr Thr Cys Ala
545                 550                 555                 560
Ala Ala Gly Ala Thr Ala Thr Ala Met Ala Thr Thr Gly Gly Gly Thr
                565                 570                 575
Ser Val Thr Val Ser Ser Ala Ile Leu Leu Thr Gly Ser Pro Ala Ser
            580                 585                 590
Leu Ala Val Ser Leu Gly Gly Ala Ala Thr Ile Ser Thr Ala Ala Ser
        595                 600                 605
Leu Ser Val Ser Thr Ser Gly Thr Ser Thr Met His Thr Ala Gly Gly
    610                 615                 620
Leu Pro Gly Pro Pro Ala Leu Leu Ile Thr Leu Val Ser Ala Leu Gly
625                 630                 635                 640
Ser Gly Val Pro Ala Ala Pro Ser Gly Ser Gly Ser Gly Thr Ala Pro
                645                 650                 655
Thr Leu Ala Ile His Pro Val Gly Gly Gly Ala Ala Ala Thr Thr Thr
            660                 665                 670
Cys Gly His Ile Ala Gly Leu Thr Ala Ser Gly Gly Gly Pro Ser Thr
        675                 680                 685
Leu Ala Gly Val Thr Leu Leu Val Ser Gly Pro Gly Ile Leu Leu Pro
    690                 695                 700
Ser Gly Thr Leu Ser Leu Thr Cys Ser Pro Ser Gly Pro Ser Leu Ser
705                 710                 715                 720
Thr Ser Gly Met Gly Val Gly Thr Ile Ala Gly Pro Ser Gly Leu Gly
                725                 730                 735
Leu Gly Thr Leu Ala His Ile Thr Thr Ala Ala Ala Leu Thr Thr Ala
            740                 745                 750
Pro Ser Leu Leu Ser Gly Leu Thr Ile Ser Leu Ala Thr Ser Ala Ala
        755                 760                 765
Gly Val Pro Leu Leu Ile Thr Ser Val Ala Thr Ala Ala Thr Ala Thr
    770                 775                 780
Thr Thr Cys Ala Ala Ala Gly Ala Thr Ala Thr Ala Met Ala Thr Thr
785                 790                 795                 800
Gly Gly Gly Thr Ser Val Thr Val Ser Ser Ala Ile Val Met Thr Gly
                805                 810                 815
Ser Pro Ala Ser Leu Ser Ala Ser Val Gly Gly Thr Val Thr Ile Thr
            820                 825                 830

Cys Ala Ala Ser Gly Ala Gly Ala Ile His Ala Thr Leu Ala Thr Thr
        835                 840                 845
Gly Gly Leu Gly Gly Leu Ser Pro Gly Leu Leu Val Thr Ala Ala Leu
    850                 855                 860
Thr Leu Ala Ala Gly Val Pro Ser Ala Pro Ser Gly Ser Gly Ser Gly
865                 870                 875                 880
Thr Gly Thr Ser Leu Leu Ile Ala Ser Leu Gly Pro Gly Ala Pro Gly
                885                 890                 895
Ser Thr Thr Cys Gly His Pro Thr Ser Thr Pro Thr Thr Pro Gly Gly
            900                 905                 910
Gly Thr Leu Leu Gly Ile Leu Gly Val Thr Leu Leu Gly Cys Gly Pro
        915                 920                 925
Gly Ile Leu Leu Pro Ser Gly Thr Leu Ser Leu Thr Cys Ser Pro Ser
    930                 935                 940
Gly Pro Ser Leu Ser Thr Ser Gly Met Gly Val Gly Thr Ile Ala Gly
945                 950                 955                 960
Pro Ser Gly Leu Gly Leu Gly Thr Leu Ala His Ile Thr Thr Ala Ala
                965                 970                 975
Ala Leu Thr Thr Ala Pro Ser Leu Leu Thr Thr Ala Pro Ser Leu Leu
            980                 985                 990
Ser Gly Leu Thr Ile Ser Leu Ala  Thr Ser Ala Ala Gly  Val Pro Leu
        995                 1000                 1005
Leu Ile  Thr Ser Val Ala Thr  Ala Ala Thr Thr Thr  Cys Ala Ala Ala
    1010                 1015                 1020
Gly  Ala Thr Ala Thr Ala  Met Ala Thr Thr Gly  Gly Gly Thr Ser Val
1025                 1030                 1035                 1040
Thr Val Ser Ser Ala  Ile Val Met Thr Gly  Ser Pro Ala Ser Leu  Ala
                1045                 1050                 1055
Val Ser Leu Gly  Gly Ala Ala Thr Ile  Ser Thr Ala Ala Ser  Leu Ser
            1060                 1065                 1070
Val Ser Thr  Ser Gly Thr Ser Thr  Met His Thr Ala Gly  Gly Leu Pro
        1075                 1080                 1085
Gly Gly  Pro Pro Pro Leu Leu  Ile Thr Leu Val Ser  Ala Leu Gly Ser
    1090                 1095                 1100
Gly  Val Pro Ala Ala Pro  Ser Gly Ser Gly Ser  Gly Thr Ala Pro Thr
1105                 1110                 1115                 1120
Leu Ala Ile His Pro  Val Gly Gly Gly Ala  Ala Ala Thr Thr Thr  Cys
                1125                 1130                 1135
Gly His Ile Ala  Gly Leu Thr Ala Ser  Gly Gly Gly Pro Ser  Thr Leu
            1140                 1145                 1150
Ala Gly Val  Leu Leu Val Cys Gly  Pro Gly Ile Leu Leu  Pro Ser Gly
        1155                 1160                 1165
Thr Leu  Ser Leu Thr Cys Ser  Pro Ser Gly Pro Ser  Leu Ser Thr Ser
    1170                 1175                 1180
Gly  Met Gly Val Gly Thr  Ile Ala Gly Pro Ser  Gly Leu Gly Leu Gly
1185                 1190                 1195                 1200
Thr Leu Ala His Ile  Thr Thr Ala Ala Ala  Leu Thr Thr Ala Pro  Ser
                1205                 1210                 1215
Leu Leu Gly Leu  Thr Ile Ser Leu Ala  Thr Ser Ala Ala Gly  Val Pro
            1220                 1225                 1230
Leu Leu Ile  Thr Ser Val Ala Thr  Ala Ala Thr Ala Thr  Thr Cys Ala
        1235                 1240                 1245
Ala Ala  Gly Ala Thr Ala Thr  Ala Met Ala Thr Thr  Gly Gly Gly Thr
    1250                 1255                 1260
Ser  Val Thr Val Ser Ser  Ala Ile Val Met Thr  Gly Ser Pro Ala Ser
1265                 1270                 1275                 1280
Leu Ala Val Ser Leu  Gly Gly Ala Ala Thr  Ile Ser Thr Ala Ser  Leu
                1285                 1290                 1295
Ser Val Ser Thr  Ser Gly Thr Ser Thr  Met His Thr Ala Gly  Gly Leu
            1300                 1305                 1310
Pro Gly Gly  Leu Pro Gly Gly Pro  Pro Pro Leu Leu Ile  Thr Leu Val

1315                    1320                    1325
Ser Ala  Leu Gly Ser Gly Val  Pro Ala Ala Pro Ser  Gly Ser Gly Ser
    1330                    1335                    1340
Gly  Thr Ala Pro Thr Leu  Ala Ile His Pro Val  Gly Gly Gly Ala Ala
1345                    1350                    1355                    1360
Ala Thr Thr Thr Cys  Gly His Ile Ala Gly  Leu Thr Ala Ser Gly  Gly
                1365                    1370                    1375
Gly Pro Ser Thr  Leu Ala
            1380

## Claims

1. Use of an agent for inhibiting the ectopic expression of a PIWIL2 gene in preparation of an anti-tumor drug.

2. Use of a protein antibody against the ectopic expression of the PIWIL2 gene in preparation of an anti-tumor drug.

3. The use of a protein antibody against the ectopic expression of the PIWIL2 gene in preparation of an anti-tumor drug according to claim 2, wherein
the protein antibody against the ectopic expression of the PIWIL2 gene is an anti-PL2L protein antibody.

4. The use of a protein antibody against the ectopic expression of the PIWIL2 gene in preparation of an anti-tumor drug according to claim 3, wherein
the anti-PL2L protein antibody comprises at least one of anti-PL2L80, anti-PL2L80A, anti-PL2L60, anti-PL2L60A, anti-PL2L50 and anti-PL2L40 protein antibodies.

5. The use of a protein antibody against the ectopic expression of the PIWIL2 gene in preparation of an anti-tumor drug according to claim 4, wherein
the anti-PL2L protein antibody is an anti-PL2L60 protein antibody.

6. The use of a protein antibody against the ectopic expression of the PIWIL2 gene in preparation of an anti-tumor drug according to claim 5, wherein
the anti-PL2L60 protein antibody is a KAO3 monoclonal antibody, and the KAO3 monoclonal antibody sequence is as shown in SEQ ID NO: 1.

7. The use of a protein antibody against the ectopic expression of the PIWIL2 gene in preparation of an anti-tumor drug according to claim 5, wherein
the anti-PL2L60 protein antibody is an antibody that inhibits STAT3 or BCL2 gene activation.

8. The use of a protein antibody against the ectopic expression of the PIWIL2 gene in preparation of an anti-tumor drug of any according to claims 2-7, wherein
the tumor comprises any one of breast cancer, lung cancer, liver cancer, bladder cancer, cervical cancer, prostate cancer, gastric cancer, leukemia, colorectal cancer, colon cancer, ovarian cancer and testicular germ cell tumors.

9. An anti-tumor pharmaceutical composition, wherein
The anti-tumor pharmaceutical composition is composed of the agent for inhibiting the ectopic expression of the PIWIL2 gene according to claim 1 and a pharmaceutically-acceptable adjuvant.

10. An anti-tumor pharmaceutical composition, wherein
the anti-tumor pharmaceutical composition is composed of the protein antibody against the ectopic expression of the PIWIL2 gene according to claim 2 and a pharmaceutically-acceptable adjuvant.

11. The anti-tumor pharmaceutical composition according to claim 9 or 10, wherein the adjuvant comprises at least one of a filler, a diluent, a wetting agent, a binder, a disintegrant, a lubricant, a film coating material, a drop pill matrix, and a condensed liquid; or
alternatively, the adjuvant comprises at least one of starch, dextrin, lactose, microcrystalline cellulose, sugar alcohol, ethanol, adhesive cement, polyethylene glycol, methyl cellulose, sodium carboxymethyl starch, magnesium stearate, fine powder silica gel, talcum powder and triethyl citrate.

12. The anti-tumor pharmaceutical composition according to claim 10, wherein the antibody is an anti-PL2L protein antibody.

13. The anti-tumor pharmaceutical composition of any according to claims 10-12, wherein the antibody comprises at least one of anti-PL2L80, anti-PL2L80A, anti-PL2L60, anti-PL2L60A, anti-PL2L50, and anti-PL2L40 protein antibodies.

14. The antibody of any according to claims 10-13, wherein the antibody is an anti-PL2L60 protein antibody.

15. The antibody according to claim 14, wherein the anti-PL2L60 protein antibody is a KAO3 monoclonal antibody, and the KAO3 monoclonal antibody sequence is as shown in SEQ ID NO: 1.

16. The antibody of any according to claims 10-12, wherein the antibody is a therapeutic antibody that targets to PL2L60 and/or inhibits activation of STAT3 and BCL2.

17. The antibody of any according to claims 10-14, wherein the amino acid sequence of the antibody comprises a sequence as shown in SEQ ID NO: 1 and/or a derived sequence which is obtained through substitutions and/or deletions and/or additions of multiple amino acid residues and has the same biological activity as SEQ ID NO: 1.

18. A method for treating a tumor, comprising: inhibiting ectopic expression of a PIWIL2 gene; and preferably inhibiting the ectopic expression of the PIWIL2 gene by a KAO3 monoclonal antibody, wherein the sequence of the KAO3 monoclonal antibody is shown as SEQ ID NO:1; and
the tumor comprises any one of breast cancer, lung cancer, liver cancer, bladder cancer, cervical cancer, prostate cancer, gastric cancer, leukemia, colorectal cancer, colon cancer, ovarian cancer and testicular germ cell tumors.
